Europäisches Patentamt

(19) European Patent Office

Office européen des brevets

(11) Numéro de publication : **0 158 659**
**B1**

(12) FASCICULE DE BREVET EUROPÉEN

(45) Date de publication du fascicule du brevet :
07.09.88

(51) Int. Cl.⁴ : **A 61 F   2/54**

(21) Numéro de dépôt : **84903552.2**

(22) Date de dépôt : **28.09.84**

(86) Numéro de dépôt international :
**PCT/FR 84/00212**

(87) Numéro de publication internationale :
**WO/8501437 (11.04.85 Gazette 85/09)**

(54) PERFECTIONNEMENTS APPORTES AUX PROTHESES TOTALES DE MAIN.

(30) Priorité : 05.10.83 FR 8315850

(43) Date de publication de la demande :
23.10.85 Bulletin 85/43

(45) Mention de la délivrance du brevet :
07.09.88 Bulletin 88/36

(84) Etats contractants désignés :
AT BE CH DE GB LI LU NL SE

(56) Documents cités :
DE-C-   177 927
DE-C-   301 803
FR-A-   484 845
FR-A-   514 600
FR-A-   514 962
FR-A- 1 324 097
US-A- 2 493 776
US-A- 2 500 614

(73) Titulaire : **MONESTIER, Jacques**
**89 Grande Rue**
**F-95760 Valmondois (FR)**

**COMPAGNIE GENERALE DE PARTICIPATION S.A.**
**(C.G.P.)**
**2, Boulevard Royal**
**Luxembourg (LU)**

(72) Inventeur : **Monestier, Jacques**
**89 Grande Rue**
**F-95760 Valmondois (FR)**

(74) Mandataire : **Orès, Bernard et al**
**Cabinet ORES 6, Avenue de Messine**
**F-75008 Paris (FR)**

## Description

La présente invention est relative à une prothèse perfectionnée de main.

On connaît déjà, par US-A-2 493 776, une prothèse totale de main comportant une coquille à plaque dorsale et à plaque palmaire entre lesquelles sont montés des doigts articulés en deux parties. Une telle prothèse n'est pas du type dit à préhension molle. Elle est en outre d'un mécanisme d'actionnement complexe, donc nécessairement lourde et d'emploi limité.

On connaît également, par FR-A-484 845 un bras artificiel avec main préhensile dans lequel les phalanges des doigts formées par de petits tubes en tôle sont actionnées par des fils en partie logés dans de petits tubes flexibles fixés aux phalanges adjacentes. Dans cette réalisation, également, les doigts sont articulés dans une coquille et ne sont donc pas aisément démontables pour des réparations éventuelles.

Dans FR-A-2 236 478 a déjà été proposée une prothèse de main dite à préhension molle et comportant une partie dorsale et une partie palmaire, la partie dorsale, en un matériau rigide approprié, comportant une plaque de base de montage des prothèses de doigts, lesdites prothèses de doigts se composant chacune, pour les quatre doigts en tenant lieu d'index, de majeur, d'annulaire et d'auriculaire, de trois éléments, constituant les phalanges, phalangines et phalangettes, juxtaposés dans le sens longitudinal, moulés pour présenter la structure des parties dorsale et latérale du doigt correspondant et articulés l'un par rapport à l'autre et par rapport à la plaque de base, tandis que la prothèse du pouce est réalisée par moulage d'une pièce unique, avec, pour chaque doigt, un câble de traction fixé à son extrémité distale l'extrémité distale de chaque phalangette.

Bien que la prothèse selon FR-A-2 236 478, soit une prothèse de main présentant une aptitude à la préhension très élaborée, elle présente cependant un certain nombre d'inconvénients dus au fait que les pièces mécaniques sont associées pour certaines (les ressorts de traction) à la partie dorsale et pour d'autres (les fils d'articulation) à la partie palmaire de la prothèse, ce qui constitue un inconvénient important au niveau de la fabrication, puisque la partie dorsale, la partie palmaire et les pièces mécaniques de la prothèse sont fabriquées séparément puis montées conjointement pour former la prothèse. Ceci constitue également un grave inconvénient dans le cas où en raison de leur usure ou d'un défaut de fonctionnement, les pièces mécaniques de la prothèse doivent être réparées ou remplacées, attendu qu'une intervention sur la prothèse en vue de la réparation ou du remplacement de pièces mécaniques intéresse les deux parties dorsale et palmaire de la prothèse et est donc compliquée et onéreuse. De plus, dans la prothèse connue, selon FR-A-2 236 478, si l'un des doigts devient défectueux et inutilisable, c'est l'ensemble du mécanisme qui doit être remplacé.

Le problème qui forme la base de la présente invention est celui de pourvoir à une prothèse de main qui répond mieux aux nécessités de la pratique que les prothèses de main proposées antérieurement, en particulier en ce que la disposition des pièces mécaniques est rationalisée pour ne les solidariser qu'à une seule partie de la prothèse, laquelle partie peut être, en cas de besoin, détachée provisoirement de l'autre partie, pour être réparée ou remplacée, les doigts de la main prothétique pouvant, quant à eux, être détachés individuellement pour être remplacés en cas d'usure, de fracture ou de défectuosité.

Ce problème est résolu, selon l'invention, par une prothèse de main dite à préhension molle, caractérisée en ce que la partie palmaire en matériau souple et la partie dorsale sont solidarisées entre elles par tout moyen détachable approprié (comme un clipsage, emboîtement, encastrement à tenons et mortaises, collage), ladite partie dorsale portant sur sa face dorsale un équilibreur de forces à fléaux asymétriques, auxquels sont attachées les extrémités des câbles, le passage de ces derniers de la face palmaire à la face dorsale étant réalisé pour chaque câble par l'intermédiaire de supports de coulisseaux portés par la plaque de base, tandis que chaque coulisseau fixé sur les phalanges, phalangines, et phalangettes est muni d'un segment de ressort de traction dans lequel coulisse le câble de traction, la longueur de chaque segment de ressort étant telle que les segments successifs présentent une discontinuité de part et d'autre d'un axe d'articulation transversal porté par la rotule correspondante d'une phalange, phalangine, ou phalangette, par rapport à ladite rotule, les extrémités voisines de deux segments de ressorts de traction successifs jouant le rôle de butée de limitation de l'angle de pliure des articulations interphalangiennes.

Selon une disposition avantageuse de ce mode de réalisation, chaque prothèse de doigt à trois phalanges articulées est pourvue d'un ressort de rappel fixé à son extrémité distale à un crochet interne prévu à l'extrémité distale de chaque phalangette, et débouchant à son extrémité opposée, sur la face dorsale, par un orifice ménagé dans chaque pièce jouant le rôle de rotule métacarpienne, lequel ressort est pourvu au voisinage de ladite extrémité opposée, d'un système de réglage approprié.

Selon une autre disposition avantageuse de ce mode de réalisation, un deuxième coulisseau est juxtaposé au premier, dans la phalange d'au moins l'un des quatre doigts précités, et guide le passage d'un second câble dans ladite phalange, lequel empêche le relèvement de ladite phalange lorsque le pouce et le doigt en question sont en opposition.

Selon encore une autre disposition avantageuse de ce mode de réalisation, chaque axe d'articulation transversal d'une phalange, phalangine ou phalangette sur la rotule correspondante de la phalange qui la précède dans le sens longitudinal, porte un tube d'entretoise qui assure un écartement uniforme entre le ressort de rappel et le plan d'articulation interphalangienne.

Selon une autre disposition avantageuse de ce mode de réalisation, la prothèse partielle dorsale de pouce est articulée pour être mise en opposition avec les autres doigts, à l'aide d'un mécanisme comportant un plateau mobile sensiblement perpendiculaire à la plaque de base, fixé à l'une de ses extrémités à la phase interne de la prothèse de pouce et dont l'extrémité opposée est prise entre deux plateaux fixes portés directement ou indirectement par la plaque de base, l'un de ces deux plateaux fixes présentant un profil de roue à rochet dans les crans de laquelle engrène un cliquet relié par un ressort à un support pivotant, et des ressorts de rappel disposés sensiblement parallèlement aux plateaux mobiles et fixes et reliant le premier à chacun des seconds, ramenant le pouce en position de repos.

Selon encore une autre disposition avantageuse de ce mode de réalisation, l'équilibreur de forces auquel sont fixées les extrémités proximales de chaque câble de traction des doigts, reçoit, en outre, l'extrémité du câble de traction unique qui commande le fonctionnement de la prothèse de main conforme à l'invention, dont l'autre extrémité est reliée à un moyen de commande physique tel que les épaules du porteur de la prothèse, par l'intermédiaire d'un harnais, ou à un moyen de commande mécanique, tel qu'un vérin à actionnement électrique, pneumatique ou hydraulique.

Selon un mode de réalisation avantageux de cet équilibreur de forces, celui-ci comprend un fléau principal sensiblement en forme d'étrier ou de U, dont chacune des extrémités porte un fléau secondaire, dont chacun reçoit les câbles de traction correspondant à deux des doigts, un oeilleton de traction solidaire du fléau principal et disposé excentré par rapport à l'axe de symétrie dudit fléau servant à l'accrochage du câble de traction qui commande le fontionnement des quatre doigts.

Conformément à l'invention, l'équilibreur de forces à fléaux asymétriques a pour effet de répartir la force unique de traction qui lui est transmise, en quatre forces décroissantes, de l'index à l'auriculaire, et de déclencher ainsi les mouvements des doigts dans l'ordre permettant la meilleure qualité de préhension (index, majeur, annulaire, auriculaire).

Selon encore une autre disposition de l'invention, la prothèse de main conforme à la présente invention est pourvue d'un système de blocage de la prothèse dans la position fermée sur un objet.

Un tel système de blocage comprend, de préférence, un corps en U dans le fond duquel passe le câble de traction et dans lequel est monté, pivotant sur un axe, un taquet coinceur, et un dispositif de commande du taquet coinceur, constitué par une vis ou par un levier excentrique pivotant sur un axe.

La mise sous contrainte du dispositif de commande provoque le serrage du taquet contre le câble de traction et son immobilisation au fond du corps en U, et la libération dudit dispositif de commande, libère le câble de commande qui coulisse alors librement dans ledit corps en U.

Conformément à l'invention, la partie palmaire de la prothèse de main est en matière souple telle qu'élastomère mousse, recouverte d'une peau et est remplaçable en cas d'usure, par séparation de la partie dorsale porteuse des pièces mécaniques, par simple démontage.

Egalement conformément à l'invention, un raccord à rotule pour le montage amovible de la prothèse de main au niveau du poignet, est fixé à l'extrémité proximale de la plaque de base.

Outre les dispositions qui précèdent, l'invention comprend encore d'autres dispositions qui ressortiront de la description qui va suivre.

La présente invention vise plus particulièrement les prothèses de main conformes aux dispositions qui précèdent, prises en elles-mêmes ou en tant qu'appareillage d'un amputé.

L'invention sera mieux comprise à l'aide du complément de description qui va suivre, qui se réfère aux dessins annexés dans lesquels :

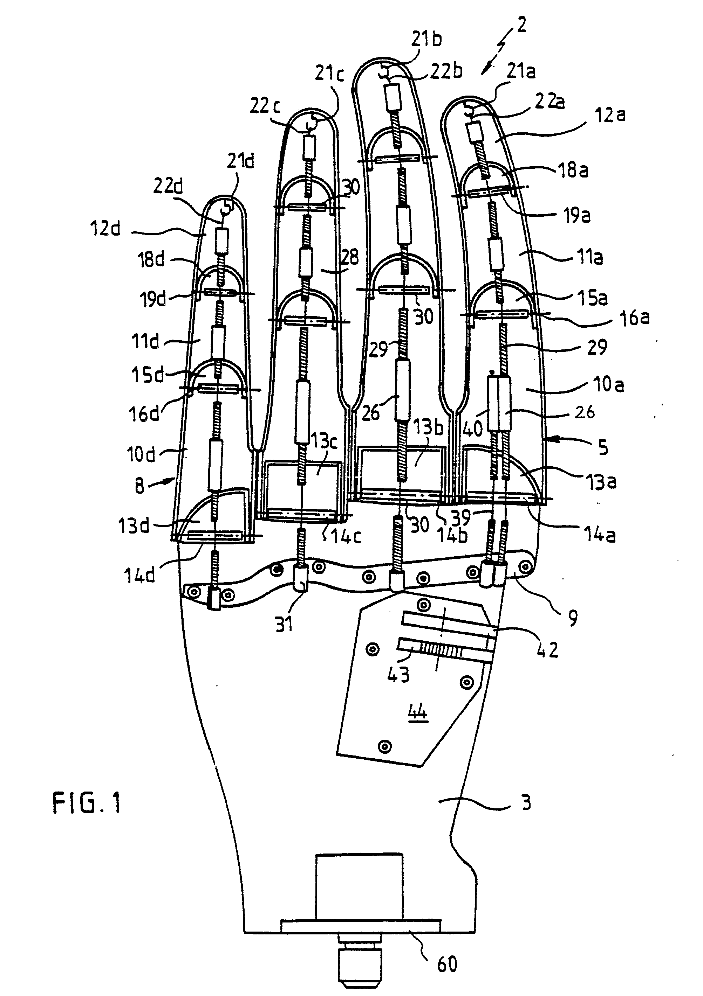

la figure 1 est une vue en plan de la face palmaire de la partie dorsale d'une prothèse de main conforme à l'invention ;

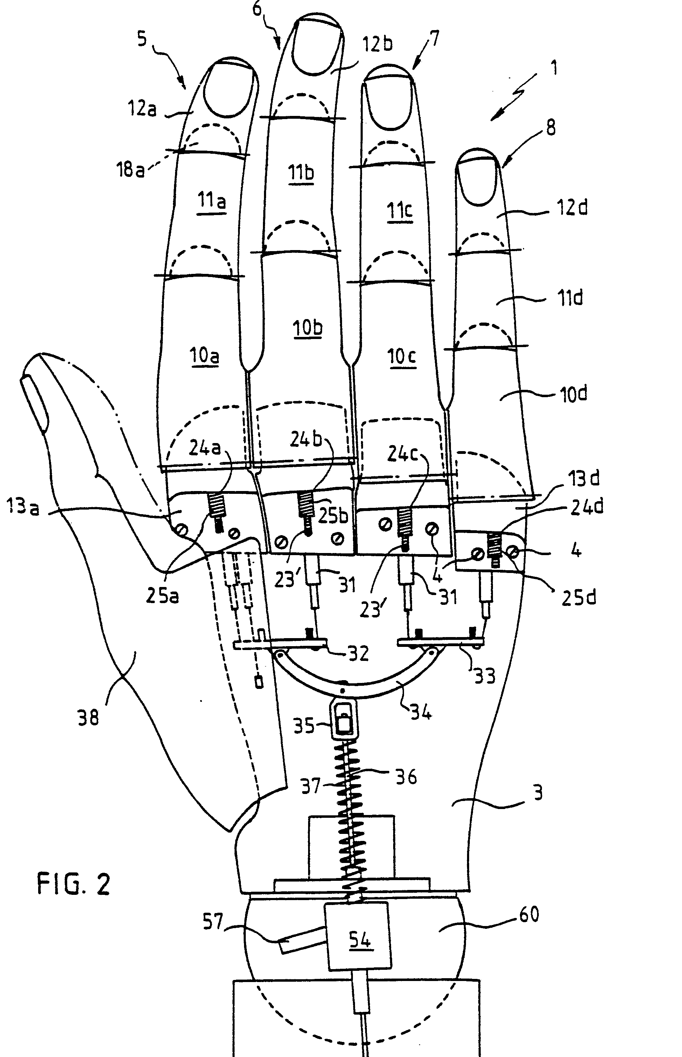

la figure 2 est une vue en plan de la face dorsale de la partie dorsale d'une prothèse de main conforme à l'invention ;

la figure 3 est une vue en coupe d'un doigt d'une prothèse conforme à l'invention ;

les figures 4a et 4b ainsi que la figure 5 représentent en coupe axiale un dispositif de blocage de la prothèse en position, et

la figure 6 est une vue de dessous du dispositif de déplacement du pouce en position d'opposition.

Il doit être bien entendu, toutefois, que ces dessins et les parties descriptives correspondantes, sont donnés uniquement à titre d'illustration de l'objet de l'invention, dont ils ne constituent en aucune manière une limitation.

La prothèse de main conforme à la présente invention, comprend une partie dorsale 1 à laquelle sont associées les pièces mécaniques d'articulation de ladite prothèse, laquelle partie dorsale 1 est réalisée par moulage, en matériau rigide à résistance mécanique élevée, tel que du métal (bronze, acier), de la matière plastique appropriée, des fibres de carbone, etc..., et une partie palmaire 2 en matière souple, notamment en caoutchouc mousse recouvert de peau, qui assure une préhension « molle » qui procure le confort à la préhension et une bonne adaptation à l'objet, souhaités par les porteurs de prothèse.

La partie dorsale 1 comprend une plaque de base 3, à la face dorsale de laquelle sont fixés, à l'aide de vis 4, quatre doigts, à savoir un index 5, un majeur 6, un annulaire 7 et un auriculaire 8. Les doigts 5 à 8 fixés à l'aide des vis 4, sont consolidés dans leur montage à l'aide d'une barrette de fixation 9 portée par la face palmaire de ladite partie dorsale 1, dans laquelle débouchent les extrémités des vis 4.

Les doigts 5 à 8 comportent chacun trois éléments juxtaposés longitudinalement, 10a-11a-12a à 10d-11d-12d qui sont articulés l'un par rapport à l'autre, comme on l'expliquera plus loin, et ces doigts sont fixés chacun à la plaque de base 3 par l'intermédiaire d'une « rotule » métacarpienne 13a à 13d fixée sur ladite plaque 3 par les vis 4. Les éléments 10a à 10d qui constituent les phalanges des doigts 5 à 8, sont montés sur la rotule métacarpienne 13a-13d au moyen d'un axe horizontal 14a-14d ; chaque phalange 10a à 10d comporte à son extrémité opposée à celle fixée à l'axe 14a-14d, une rotule 15a-15d dans laquelle est monté un axe 16a-16d d'articulation de l'élément 11a-11d qui constitue la phalangine de chaque doigt 5-8, et qui se termine à son extrémité opposée, distale, par une rotule 18a-18d, qui porte à son tour un axe 19a-19d d'articulation de l'élément 12a-12d qui constitue la phalangette de chaque doigt 5-8. Chacune de ces phalangettes 12a-12d comporte à son extrémité distale interne, c'est-à-dire sur sa face palmaire, un crochet 21a-21d auquel sont fixés d'une part un câble de traction 22a-22d et d'autre part un ressort de rappel 23a-23d ; selon une variante, on peut prévoir deux crochets séparés pour la fixation du câble de traction et du ressort de rappel, respectivement.

Le ressort de rappel 23 débouche, au voisinage de son extrémité opposée 23', sur la face dorsale de ladite partie dorsale 1 de la prothèse de main conforme à l'invention, par un orifice 24a-24d ménagé dans la « rotule » métacarpienne 13a-13d ; ce ressort de rappel 23 est pourvu d'un système de réglage 25a-25d.

Le câble de traction 22a-22d est guidé dans chaque doigt 5-8 par des coulisseaux 26 tubulaires montés chacun sur un support 27 venu de moulage avec la partie dorsale 1 ou soudé sur le fond 28 des doigts 5-8. Un coulisseau tubulaire 26 supporté par un support 27 est prévu en association avec chacune des phalanges, phalangines et phalangettes. Le câble de traction 22a-22d coulisse en outre dans des segments de ressorts de traction 29 sertis dans les coulisseaux tubulaires 26 de façon telle qu'en position de repos de la prothèse, le câble de traction est libre de tout guidage, dans une zone 30, qui correspond à la zone des rotules d'articulation 13a-13d, 15a-15d et 18a-18d. Chaque câble 22a-22d traverse la plaque de base 3 par l'intermédiaire de supports de coulisseaux 31 soudés sur la plaque de base 3 ou venus de moulage avec cette dernière, afin d'aboutir sur la face dorsale de la partie dorsale 1 de la prothèse, où chaque câble 22a-22d est fixé aux fléaux asymétriques secondaires 32-33 d'un équilibreur de forces, eux-mêmes portés par un fléau principal 34 qui porte, excentré par rapport à son axe de symétrie, un œilleton 35 d'accrochage du câble de traction 36 qui commande l'articulation des quatre doigts 5-8 sous l'effet d'une force de traction unique communiquée par des mouvements des épaules exécutés par le porteur de la prothèse, elle-même reliée aux épaules par un harnais, ou par un vérin électrique, pneumatique ou hydraulique (non représentés). Un ressort de compression 37 qui entoure le câble de traction 36 neutralise les frottements de ce dernier dans la gaine.

L'œilleton 35 est excentré en direction du pouce 38, de manière à répartir la force unique de traction communiquée par le câble 36, en quatre forces décroissantes, de l'index à l'auriculaire, et de déclencher ainsi le mouvement des doigts dans l'ordre permettant la meilleure qualité de préhension, soit : index, majeur, annulaire, auriculaire.

Chacun des axes 14a-14d, 16a-16d et 18a-18d d'articulation des phalanges, phalangines et phalangettes entre elles comporte un petit tube d'entretoise qui assure un écartement uniforme du ressort de rappel 23a-23d par rapport au plan d'articulation interphalangienne.

L'index 5 comporte un câble de traction supplémentaire 39 guidé dans un coulisseau 40 juxtaposé au coulisseau 26 et solidaire, de même que ce dernier, du fond de la phalange 10a, lequel câble 39 empêche la phalange 10a de se relever lorsque le pouce 38 et l'index 5 sont en opposition.

Le pouce 38 est mû par un mécanisme qui permet de le mettre dans différentes positions en opposition par rapport aux autres doigts. Ce mécanisme comporte un plateau 41 sensiblement perpendiculaire à la plaque de base 3, et rendu solidaire du pouce 38 par des vis (non représentées). A son extrémité opposée à celle qui est solidaire du pouce, le plateau 41 est pris entre deux plateaux 42-43 qui se trouvent dans des plans parallèles au plan dudit plateau 41. Ces plateaux 42-43 sont fixés à la plaque de base 3 soit directement (par soudage ou venus de moulage), soit indirectement par l'intermédiaire d'une platine 44. Le plateau 41, qui est mobile, est fixé sur un axe 45 entre les plateaux fixes 42-43, dont le plateau inférieur 43 présente un profil en forme de roue à rochet dans les crans 46 de laquelle vient engrener un cliquet 47 fixé sur la face inférieure du plateau 41 et relié par un ressort 48 à un support 49 pivotant, l'engrènement du cliquet 47 avec l'un ou l'autre des crans 46 amenant le pouce à volonté en opposition avec l'un quelconque des quatre autres doigts. Deux ressorts de rappel 50 fixés d'une part sur deux ergots 52 de la platine 44 (ou de la plaque de base 3) et d'autre part sur deux ergots 53 du plateau mobile 41 rappellent le pouce 38 en position de repos.

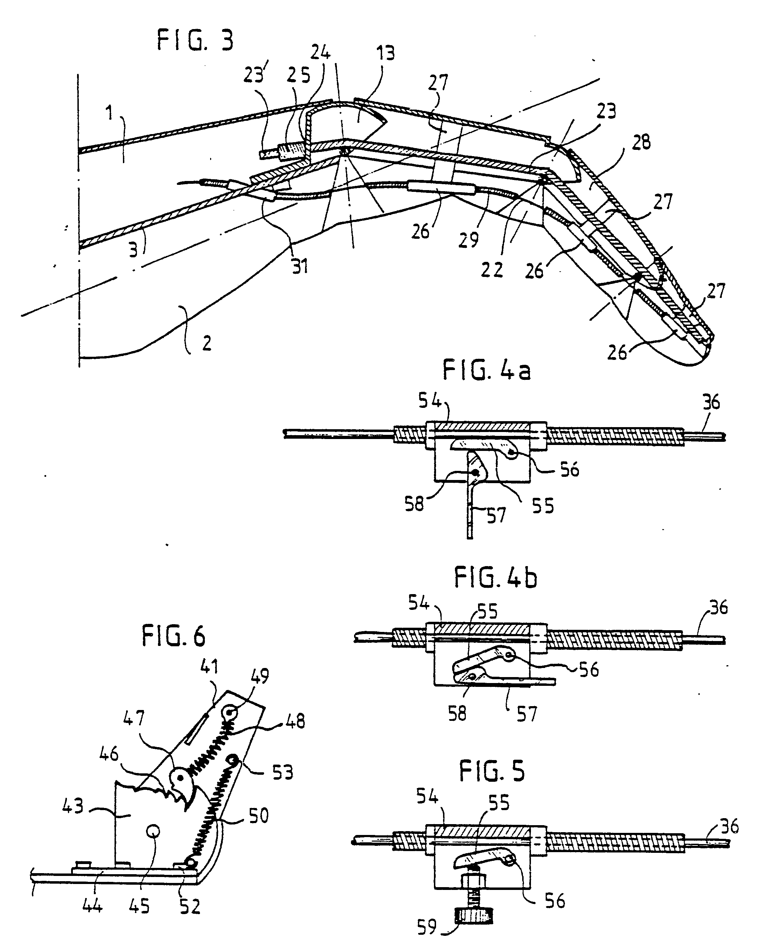

Un système de blocage permet de bloquer la prothèse dans la position fermée sur un objet (par exemple sur un stylo pour écrire) ; selon le mode de réalisation représenté aux figures 4a et 4b et 5, le système comprend un corps en U 54 dans le fond duquel coulisse le câble de traction 36 ; un taquet coinceur 55 pivotant autour d'un axe 56 entre les ailes du U ; un organe de blocage tel que le levier excentrique 57 pivotant autour de l'axe 58 entre les ailes du U (cf. figures 4a et 4b) ou tel qu'une vis de blocage 59 (cf. figure 5) provoque le serrage du taquet 55 contre le câble de traction 36, qui est bloqué sur le fond du corps en U 54. Le déblocage du câble 36 a lieu par dégagement du taquet 55 par actionnement du levier 57 ou desserrage de la vis 59.

Un raccord 60 à rotule pour le montage de la prothèse au niveau du poignet est fixé, à l'aide de vis, à la plaque de base 3 et le système de blocage 54-58 ou 59 peut avantageusement être fixé audit raccord 60.

Pour des raisons esthétiques, la plaque de base, le raccord 60 à rotule et l'équilibreur de forces sont dissimulés par un capot fixé à la plaque de base 3 et aux doigts de la prothèse, sur lesquels il peut être articulé.

L'actionnement des câbles d'articulation 22a-22d provoque l'articulation des phalanges les unes par rapport aux autres, l'angle de pliure des articulations interphalangiennes étant cependant limité par l'effet de butée réciproque exercé par l'entrée en contact des extrémités qui se font face de deux segments de ressort de traction 29.

La fixation des doigts par des vis permet leur remplacement en cas de besoin, de même que le montage des parties palmaire 2 et dorsale 1 par emboîtement ou analogue l'une dans l'autre permet le remplacement rapide de l'une ou l'autre de ces parties, sans difficulté et sans l'intervention d'un prothésiste.

La conception de la prothèse de main conforme à la présente invention permet :

— sa fabrication à l'échelle industrielle

— sa réparation, dans des conditions considérablement simplifiées, ne requérant pas l'intervention d'un spécialiste ;

— l'appareillage d'amputés à moignon long, en conservant la supination naturelle, grâce au fait que tous les organes mécaniques sont logés à l'intérieur de la prothèse de main, et la commande, pour les mêmes raisons, à l'aide d'un seul câble de commande.

Bien que la prothèse de main conforme à l'invention, n'ait été décrite dans ce qui précède que dans son utilisation prothétique, l'on comprendra aisément qu'elle peut trouver une utilisation particulièrement intéressante en robotique, où, placée au bout d'un bras robotisé, elle peut être utilisée de façon très étendue en raison de sa grande précision de préhension, permettant, en particulier, son utilisation pour la préhension et le classement d'objets placés en vrac et de forme indéterminée, pour la manipulation d'objets lourds et volumineux, notamment dans le domaine de la construction à partir d'éléments préfabriqués, son utilisation pour la manipulation télécommandée d'objets en milieu hostile, son utilisation sur des vaisseaux et satellites spatiaux ou des soucoupes plongeantes, etc..., ses utilisations médicales et industrielles n'excluant pas son utilisation pour la réalisation d'automates ou d'autres oeuvres d'art similaires.

Ainsi que cela ressort de ce qui précède, l'invention ne se limite nullement à ceux de ses modes de réalisation et d'application qui viennent d'être décrits de façon plus explicite.

## Revendications

1. Prothèse de main dite à préhension molle, comportant une partie dorsale (1) et une partie palmaire (2), la partie dorsale (1), en un matériau rigide approprié comportant une plaque de base (3) de montage des prothèses de doigts (5-8), lesdites prothèses de doigts se composant chacune pour les quatre doigts tenant lieu d'index (5), de majeur (6), d'annulaire (7) et d'auriculaire (8), de trois éléments (10a-11a-12a à 10d-11d-12d), constituant les phalanges (10a-10d), phalangines (11a-11d) et phalangettes (12a-12d), juxtaposés dans le sens longitudinal, moulés pour présenter la structure des parties dorsale et latérale du doigt correspondant et articulés l'un par rapport à l'autre et par rapport à la plaque de base (3) autour de « rotules » (13a-13d, 15a-15d, 18a-18d), tandis que la prothèse du pouce (38) est réalisée par moulage d'une pièce unique, avec, pour chaque doigt, un câble de traction (22a-22d) fixé à son extrémité distale à l'extrémité distale de chaque phalangette (12a-12d), lesdits câbles étant guidés sur une partie de leur longueur par des coulisseaux (26) fixés au fond de la face palmaire de chacun des éléments caractérisée en ce que la partie palmaire (2) en matériau souple et la partie dorsale (1) sont solidarisées entre elles par tout moyen détachable approprié (comme un clipsage, emboîtement, encastrement à tenons et mortaises, collage), ladite partie dorsale (1) portant sur sa face dorsale un équilibreur de forces à fléaux asymétriques (32, 33) auxquels sont attachées les extrémités des câbles (22a-22d), le passage de ces derniers de la face palmaire à la face dorsale étant réalisé pour chaque câble par l'intermédiaire de supports de coulisseaux (31) portés par la plaque de base (3), tandis que chaque coulisseau (26) fixé sur les phalanges, phalangines et phalangettes est muni d'un segment (29) de ressort de traction dans lequel coulisse le câble de traction (22a-22d), la longueur de chaque segment de ressort étant telle que les segments successifs présentent une discontinuité de part et d'autre d'un axe d'articulation transversal (14-16-19) porté par la rotule correspondante, d'une phalange, phalangine ou phalangette, par rapport à ladite rotule, les extrémités voisines de deux segments de ressorts de traction successifs jouant le rôle de butée de limitation de l'angle de pliure des articulations interphalangiennes.

2. Prothèse de main selon la revendication 1, caractérisée en ce que chaque prothèse de doigt à trois phalanges articulées est pourvue d'un ressort de rappel (23a-23d) fixé à son extrémité distale à un crochet interne prévu à l'extrémité distale de chaque phalangette, et débouchant à son extrémité opposée, sur la face dorsale, par un orifice (24a-24d) ménagé dans chaque pièce (13a-13d) jouant le rôle de rotule métacarpienne, lequel ressort (23a-23d) est pourvu au voisinage de ladite extrémité opposée, d'un système de réglage (25a-25d) approprié.

3. Prothèse de main selon l'une des revendications 1 et 2, caractérisée en ce qu'un deuxième coulisseau (40) est juxtaposé au premier (26), dans la phalange (10a-10d) d'au moins l'un des quatre doigts précités, et guide le passage d'un second câble (39) dans ladite phalange, lequel empêche le relèvement de ladite phalange lorsque le pouce et le doigt en question sont en opposition.

4. Prothèse de main selon l'une quelconque des revendications 1 à 3, caractérisée en ce que chaque axe d'articulation transversal (14-16-19) d'une phalange, phalangine ou phalangette sur la rotule (13-15-18) correspondante de la phalange qui la précède dans le sens longitudinal, porte un tube d'entretoise qui assure un écartement uniforme entre le ressort de rappel (23a-23d) et le plan d'articulation interphalangienne.

5. Prothèse de main selon l'une quelconque des revendications 1 à 4, caractérisée en ce que la prothèse partielle dorsale de pouce (38) est articulée pour être mise en opposition avec les autres doigts, à l'aide d'un mécanisme comportant un plateau mobile (41) sensiblement perpendiculaire à la plaque de base (3), fixé à l'une de ses extrémités à la face interne de la prothèse de pouce et dont l'extrémité opposée est prise entre deux plateaux fixes (42-43) portés directement ou indirectement par la plaque de base (3), l'un de ces deux plateaux fixes (43) présentant un profil de roue à rochet dans les crans (46) de laquelle engrène un cliquet (47) relié par un ressort (48) à un support pivotant (49), et des ressorts de rappel (50) disposés sensiblement parallèlement aux plateaux mobile (41) et fixes (42-43) et reliant le premier à chacun des seconds, ramenant le pouce en position de repos.

6. Prothèse de main selon l'une quelconque des revendications 1 à 5, caractérisée en ce que l'équilibreur de forces auquel sont fixées les extrémités proximales de chaque câble de traction (22a-22d) des doigts, reçoit, en outre, l'extrémité du câble de traction (36) unique qui commande le fonctionnement de la prothèse de main, dont l'autre extrémité est reliée à un moyen de commande physique tel que les épaules du porteur de la prothèse par l'intermédiaire d'un harnais, ou à un moyen de commande mécanique, tel qu'un vérin à actionnement électrique, pneumatique ou hydraulique.

7. Prothèse de main selon la revendication 6, caractérisée en ce que l'équilibreur de forces à fléaux asymétriques comprend un fléau principal (34) sensiblement en forme d'étrier ou de U, dont chacune des extrémités porte un fléau secondaire (32-33), dont chacun reçoit les câbles de traction (22a-22d) correspondant à deux des doigts, un oeilleton (35) de traction solidaire du fléau principal et disposé excentré par rapport à l'axe de symétrie dudit fléau servant à l'accrochage du câble de traction qui commande le fonctionnement des quatre doigts.

8. Prothèse de main selon l'une quelconque des revendications 1 à 7, caractérisée en ce qu'elle est pourvue d'un système de blocage de la prothèse dans la position fermée sur un objet.

9. Prothèse de main selon la revendication 8, caractérisée en ce que ledit système de blocage comprend un corps en U (54) dans le fond duquel passe le câble de traction (36) et dans lequel est monté, pivotant sur un axe, un taquet coinceur (55), et un dispositif de commande du taquet coinceur, constitué par une vis (59) ou par un levier excentrique (57) pivotant sur un axe (58) dont la mise sous contrainte provoque le serrage du taquet (55) contre le câble de traction (36) et son immobilisation au fond du corps en U (54), et la libération dudit dispositif de commande, libère le câble de commande qui coulisse alors librement dans ledit corps en U.

10. Prothèse de main selon l'une quelconque des revendications 1 à 9, caractérisée en ce que sa partie palmaire (2) est en matière souple telle qu'élastomère mousse, recouverte d'une peau et est remplaçable en cas d'usure, par séparation de la partie dorsale porteuse des pièces mécaniques, par simple démontage.

11. Prothèse de main selon l'une quelconque des revendications 1 à 10, caractérisée en ce qu'un raccord (60) à rotule pour le montage amovible de la prothèse de main au niveau du poignet, est fixé à l'extrémité proximale de la plaque de base.

12. Application de la prothèse de main selon l'une quelconque des revendications 1 à 11, dans le domaine de la robotique.

**Claims**

1. A so called hand prosthesis, comprising a dorsal part (1) and a palmar part (2), the dorsal part (1), made from an appropriate rigid material, comprising a base plate (3) for mounting the finger prostheses (5-8) said finger prostheses each being formed, for the four fingers serving as forefinger (5), second finger (6), third finger (7) and little finger (8) of three elements (10a-11a-12a to 10d-11d-12d), forming the phalanges (10a-10g), the middle phalanges (11a-11d) and the end phalanges (12a-12d), juxtaposed in the longitudinal direction and molded so as to have the structure of the dorsal and lateral parts of the corresponding finger and articulated with respect to each other and with respect to the base plate (3) about « swivel joints » (13a-13d, 15a-15d, 18a-18d, whereas the prosthesis of the thumb (38) is formed by molding in a single piece with, for each finger, a traction cable (22a-22d) fixed at its distal end to the distal end of each end phalange (12a-12d), said cables being guided over a part of their length by slides (26) fixed to the bottom of the palmar face of each of the elements, characterized in that the palmar part (2) made from a flexible material and the dorsal part (1) are secured together by any appropriate detachable means (such as clipping, jointingly fitting, tenon and mortice jointing, bonding), said dorsal part (1) having on its dorsal face a force balancer with asymmetric arms (32, 33) to which the ends of the cables (22a-22d) are attached, the passage of these latter from the palmar face to the dorsal face being achieved for each cable by means of slide supports (31) carried by the base plate (3), whereas each slide (26) fixed to the phalanges, middle phalanges and end phalanges is provided with a traction spring segment (29) in which the traction cable (22a-22d) slides, the length of each spring segment being such that the successive segments have a discontinuity on each side of a transverse articulation axis (14-16-19) carried by the corresponding swivel joint of a phalange, middle phalange or end phalange, with respect to said swivel joint, the adjacent ends of two successive traction spring segments playing the role of a stop limiting the bending angle of the interphalange articulations.

2. Hand prosthesis according to claim 1, characterized in that each finger prosthesis with three articulated phalanges is provided with a return spring (23a-23d) fixed at its distal end to an internal hook provided at the distal end of each end phalange, and emerging at its opposite end, in the dorsal face, through an orifice (24a-24d) formed in each piece (13a-13d) playing the role of metarcarpial joint, which spring (23a-23d) is provided in the vicinity of said opposite end with an appropriate adjustment system (25a-25d).

3. Hand prosthesis according to one of claims 1 and 2, characterized in that a second slide (40) is juxtaposed to the first one (26) in the phalange (10a-10d) of at least one of said four fingers, and guides the passage of a second cable (39) into said phalange, which prevents said phalange from rising when the thumb and the finger in question are in opposition.

4. Hand prosthesis according to any one of claims 1 to 3, characterized in that each transverse hinge pin (14-16-19) of a phalange, middle or end phalange on the corresponding swivel joint (13-15-18) of the phalange which precedes it in the longitudinal direction, has a spacer tube which provides uniform spacing between the return spring (23a-23d) and the interphalange articulation plane.

5. Hand prosthesis according to any one of claims 1 to 4, characterized in that the partial dorsal thumb prosthesis (38) is articulated so as to be placed in opposition with the other fingers, by means of a mechanism comprising a mobile plate (41) substantially perpendicular to the base plate (3), fixed at one of its ends to the internal face of the thumb prosthesis and whose opposite end is sandwiched between two fixed plates (42-43) supported directly or indirectly by the base plate (3), one of these fixed plates (43) having a ratchet wheel profile in the notches (46) of which is engaged a pawl (47) connected by a spring (48) to a pivoting support (49) and return springs (50) disposed substantially parallel to the mobile (41) and fixed (42-43) plates and connecting the first to each of the second ones, bringing the thumb back to the rest position.

6. Hand prosthesis according to any one of claims 1 to 5, characterized in that the force balancer to which the proximal ends ot each traction cable (22a-22d) of the fingers are fixed further receives the end of the single traction cable (36) which controls the operation of the hand prosthesis, whose other end is connected to a physical control means such as the shoulders of the bearer of the prosthesis by means of a harness, or to a mechanical control means, such as an electric, pneumatic or hydraulic actuating jack.

7. Hand prosthesis according to claim 6, characterized in that the asymmetric arm force balancer includes a main arm (34) substantially in the form of a stirrup or U, each of the ends of which carries a secondary arm (32-33) each of which receives the traction cables (22a-22d) corresponding to two of the fingers, a traction eyelet (35) fixed to the main arm and offcentered with respect to the axis of symmetry of said arm serving for coupling the traction cable which controls the operation of the four fingers.

8. Hand prosthesis according to any one of claims 1 to 7, characterized in that it is provided with a system for locking the prosthesis in the position closed on an object.

9. Hand prosthesis according to claim 8, characterized in that said locking system comprises a U shaped body (54) on the bottom of which the traction cable (36) passes and in which is mounted, pivoting on a pin, a jamming lever (55), and a device for controlling the jamming lever formed by a screw (59) or by an excentric lever (57) pivoting on a pin (58) which, when actuated, causes the lever (55) to be clamped against the traction cable (36-) for immobilizing it at the bottom of the U shaped body (54), and release of said control device frees the control cable which then slides freely in said U shaped body.

10. Hand prosthesis according to any one of claims 1 to 9, characterized in that its palmar part (2) is made from a flexible material such as elastomer foam, covered with a skin and is replaced in the case of wear, by separation from the dorsal part carrying the mechanical pieces, by simple removal.

11. Hand prosthesis according to any one of claims 1 to 10, characterized in that a swivel connection (60) for removably fitting the hand prosthesis at the level of the wrist, is fixed to the proximal end of the base plate.

12. Application of the hand prosthesis according to any one of claims 1 to 11, in the robotics field.

**Patentansprüche**

1. Handprothese mit weichem Fassen, umfassend einen dorsalen Teil (1) und einen palmaren Teil (2), wobei der dorsale Teil (1) aus einem geeigneten steifen Material eine Basisplatte (3) für die Montage von Prothesen der Finger (5-8) umfaßt, wobei jede dieser Prothesen der Finger für die vier Finger, welche den Zeigefinger (5), den Mittelfinger (6), den Ringfinger (7) und den kleinen Finger (6) ersetzen, aus drei Elementen (10a-11a-12a bis 10d-11d-12d) besteht, welche die Phalangen (10a-10d), Mittelglieder (11a-11d) und Endglieder (12a-12d) bilden, und zwar nebeneinander in dem Longitudinalsinn, geformt, um die Struktur der dorsalen und seitlichen Teile des entsprechenden Fingers aufzuweisen und eines mit Bezug auf das andere und mit Bezug auf die Basisplatte (3) gelenkig um « Knochengelenke » (13a-13d, 15a-15d, 18a-18d) angebracht, während die Prothese des Daumens (38) durch Formung eines einzigen Teils realisiert ist, mit einem Zugkabel (22a-22d) für jeden Finger, das an seinem distalen Ende an dem distalen Ende jedes Endglieds (12a-12d) befestigt ist, wobei diese Kabel über einen Teil ihrer Länge durch Führungen (26) geführt sind, die am Boden der palmaren Seite jedes der Elemente befestigt sind, dadurch gekennzeichnet, daß der palmare Teil (2) aus elastischem Material und der dorsale Teil (1) durch jedes geeignete lösbare Mittel (wie eine Clipsverbindung, Einfügung, Einbau mit Zapfen und Zapfenlöchern, Verklebung) miteinander fest verbunden sind, wobei der dorsale Teil (1) auf seiner dorsalen Seite einen Kräteausgleicher mit asymmetrischen Waagebalken (32, 33) trägt, an denen die Enden der Kabel (22a-22d) befestigt sind, wobei der Durchgang dieser letzteren von der palmaren Seite zu der dorsalen Seite für jedes Kabel mittels Trägern von Führungen (31) realisiert ist, die von der Basisplatte (3) getragen sind, während jede Führung (26), die auf den Phalangen, Mittelgliedern und Endgliedern befestigt ist, mit einem Abschnitt (29) einer Zugfeder versehen ist, in welcher das Zugkabel (22a-22d) geführt gleitet wobei die Länge jedes Federabschnitts wie diejenige der folgenden Abschnitte ist, die eine Diskontinuität beiderseits einer querverlaufenden Gelenkachse (14-16-19), welche von dem entsprechenden Knochengelenk einer Phalanx, eines Mittelglieds oder eines Endglieds getragen ist, mit Bezug auf dieses Knochengelenk aufweisen, wobei die benachbarten Enden der beiden aufeinanderfolgenden Zugfederabschnitte die Funktion von Beschränkungsanschlägen für den Winkel des Einschlags der Schwenkbarkeit zwischen den Gliedern haben.

2. Handprothese nach Anspruch 1, dadurch gekennzeichnet, daß jede Fingerprothese mit dreigelenkig angebrachten Gliedern mit einer Rückholfeder (23a-23d) versehen ist, die an ihrem distalen Ende an einem inneren Haken befestigt ist, der an dem distalen Ende jedes Endglieds vorgesehen ist, und die an ihrem entgegengesetzten Ende auf der dorsalen Seite herauskommt, und zwar durch eine Öffnung (24a-24d), die in jedem Teil (13a-13d) angeordnet ist, welches, die Funktion des Mittelhandknochengelenks hat, wobei diese Feder (23a-23d) in der Nähe des besagten entgegengesetzten Endes mit einem geeigneten Regulierungssystem (25a-25d) versehen ist.

3. Handprothese nach einem der Ansprüche 1 und 2, dadurch gekenzeichnet, daß eine zweite Führung (40) neben der ersten (26) in der Phalanx (10a-10d) von wenigstens einem der vier vorgenannten Finger angeordnet ist und den Durchgang eines zweiten Kabels (39) in der erwähnten Phalanx führt, welches das Sichwieder-Aufrichten der besagten Phalanx verhindert, wenn der Daumen und der in Frage stehende Finger in Gegenüberstellung sind.

4. Handprothese nach irgendeinem der Ansprüche 1 bis 3, dadurch gekennzeichnet, daß jede querverlaufende Gelenkachse (14-16-19) eines Phalanx, Mittelglieds oder Endglieds auf dem entsprechenden Knochengelenk (13-15-18) des Glieds, welches im Longitudinalsinn vorhergeht, ein Verstrebungsrohr trägt, das eine gleichförmige Entfernung zwischen der Rückholfeder (23a-23d) und der Gelenkebene zwischen den Gliedern sicherstellt.

5. Handprothese nach irgendeinem der Ansprüche 1 bis 4, dadurch gekennzeichnet, daß die partielle dorsale Prothese des Daumens (38) gelenkig angebracht ist, um in Gegenüberstellung mit den anderen Fingern gebracht zu werden, und zwar mit Hilfe eines Mechanismus, der eine bewegliche Platte (41) umfaßt, die im wesentlichen senkrecht zu der Basisplatte (3) ist, und zwar befestigt an einer ihrer Enden an der Innenseite der Prothese des Daumens und deren entgegengesetztes Ende zwischen zwei feste Platten (42-43) genommen ist, die direkt oder indirekt durch die Basisplatte (3) getragen sind, wobei die eine dieser beiden festen Platten (43) ein Profil eines Schaltrads aufweist, in dessen Einschnitte eine Feststellklinke (47) einrückt, die durch eine Feder (48) mit einem drehbaren Träger (49) verbunden ist, und daß Rückholfedern (50) in wesentlichen parallel zu der beweglichen Platte (41) und den festen Platten (42-43) angeordnet sind, und die erstere mit jeder der zweiten verbinden, so daß sie den Daumen wieder in die Ruheposition bringen.

6. Handprothese nach irgendeinem der Ansprüche 1 bis 5, dadurch gekennzeichnet, daß der Kräfte ausgleicher, an dem die proximalen Enden jedes Zugkabels (22a-22d) der Finger befestigt sind, außerdem das Ende des einzigen Zugkabels (36) empfängt, welches das Funktionieren der Handprothese steuert, und dessen anderes Ende mit einem Mittel des physischen Steuerns verbunden ist, wie den. Schultern des Trägers der Prothese, und zwar mittels eines Geschirrs, oder mit einem Mittel zum mechanischen Steuern, wie einer Hebevorrichtung mit elektrischer, pneumatischer oder hydraulischer Betätigung.

7. Handprothese nach Anspruch 6, dadurch gekennzeichnet, daß der Kräfteausgleicher mit asymmetrischen Waagebalken einen Hauptwaagebalken (34) umfaßt, der im wesentlichen die Form eines Stellbügels oder eines U hat, bei dem jedes Ende einen sekundären Waagebalken (32-33) trägt, von dem jeder die Zugkabel (22a-22d) empfängt, welche zwei Fingern entsprechen, sowie eine Zugöse (35), die fest mit dem Hauptwaagebalken verbunden und exzentrisch bezüglich der Symmetrieachse dieses Waagebalkens angeordnet ist, welche zur Aufhängung des Zugkabels dient, das das Funktionieren der vier Finger steuert.

8. Handprothese nach irgendeinem der Ansprüche 1 bis 7, dadurch gekennzeichnet, daß sie mit einem System zur Blockierung der Prothese in der um ein Objekt geschlossenen Position versehen ist.

9. Handprothese nach Anspruch 8, dadurch gekennzeichnet, daß das besagte System der Blockierung einen U-Körper (54) umfaßt, in dessen Boden das Zugkabel (36) hindurchgeht und in dem drehbar um eine Achse, ein Verklemmdaumen (55) montiert ist, und eine Steuereinrichtung für den Verklemmdaumen, die von einer Schraube (59) oder von einem exzentrischen Hebel (57), der um eine Achse (58) drehbar ist, gebildet ist, deren bzw. dessen Unterbelastungsetzen die Verklemmung des Daumens (55) gegen das Zugkabel (36) und seine Unbeweglichmachung auf dem Boden des U-Körpers (54) hervorruft, wobei die Freisetzung dieser Steuervorrichtung das Steuerkabel freisetzt, das dann frei in dem U-Körper geführt gleitet.

10. Handprothese nach irgendeinem der Ansprüche 1 bis 9, dadurch gekennzeichnet, daß ihr palmarer Teil (2) aus einem elastischen Material, wie Elastomerschaum, ist, das mit einer Haut bedeckt ist und im Fall des Verschleißes ersetzbar ist, und zwar durch Trennung des dorsalen Teils, der die mechanischen Teile trägt mittels einfacher Demontage.

11. Handprothese nach irgendeinem der Ansprüche 1 bis 10, dadurch gekennzeichnet, daß eine Verbindung (60) mit Kugelgelenk für die abnehmbare Montage der Handprothese auf dem Niveau des Handgelenks an dem proximalen Ende der Basisplatte befestigt ist.

12. Anwendung der Handprothese nach irgendeinem der Ansprüche 1 bis 11 auf dem Robotergebiet.

FIG.1

FIG. 2

0 158 659

FIG. 3

FIG. 4a

FIG. 4b

FIG. 6

FIG. 5